# EUROPÄISCHE PATENTSCHRIFT

(11) **EP 1 098 600 B1**
(45) Veröffentlichungstag und Bekanntmachung des Hinweises auf die Patenterteilung: **03.03.2004**
(21) Anmeldenummer: 99939389.5
(22) Anmeldetag: 20.07.1999
(51) Int. Cl.: A61B 17/68

(54) **FIXATIONSELEMENT FÜR KNOCHENFRAGMENTE**
FIXING ELEMENT FOR BONE FRAGMENTS
ELEMENT DE FIXATION POUR FRAGMENTS OSSEUX

(30) Priorität: 25.07.1998 DE 19835096
(43) Veröffentlichungstag der Anmeldung: 16.05.2001
(73) Patentinhaber: Lob, Helke, 81377 München (DE)
(72) Erfinder: Lob, Helke, 81377 München (DE)
(74) Vertreter: Eisenführ, Speiser & Partner
(86) Internationale Anmeldenummer: PCT/EP1999/005194
(87) Internationale Veröffentlichungsnummer: WO 2000/006037

(56) Entgegenhaltungen:
- EP-A- 0 409 364
- DE-A- 4 444 510
- US-A- 4 790 304
- US-A- 5 437 674
- US-A- 5 713 904

## Beschreibung

Die Erfindung betrifft ein Fixationselement gemäß dem Oberbegriff des Anspruchs 1.

Bei überlastungsbedingten Frakturen stellt sich häufig das Problem, daß relativ kleine, exponierte Knochenbereiche vom restlichen Knochen abgetrennt werden. So stellen beispielsweise Knöchelfrakturen die häufigste Bruchverletzung der unteren Extremität dar. Hierbei wird durch Überlastung im Bereich des oberen Sprunggelenkes der Innenknöchel von der Tibia und/oder der Außenknöchel von der Fibula abgetrennt. Um die Funktion des Sprunggelenkes wiederherzustellen, müssen die Knöchelfragmente bis zur Heilung des Bruches an dem zugehörigen Knochenfragment fixiert werden. Hierzu werden noch häufig Knochenschrauben und dergleichen verwendet. Da zur ausreichenden Stabilisierung der Knochenfragmente jedoch aufgrund der Schlankheit der Knochenschrauben meist besonders lange und/oder mehrere Knochenschrauben erforderlich sind, gewinnen die einfacher und schneller zu implantierenden gattungsgemäßen Fixationselemente zunehmend an Bedeutung.

Aus der europäischen Patentanmeldung EP 0 409 364 A2 ist ein Fixationselement bekannt, bei dem der Fixationskörper an seinem proximalen, d. h. dem Operateur zugewandten Ende einen Absatz aufweist, der ein Einführen des Fixationskörpers in die Aufnahmebohrungen in den Knochenfragmenten nur bis zu einer bestimmten Tiefe ermöglicht. Der Fixationskörper, der im Ausgangszustand einen konischen Grundkörper mit auf diesem angeordneten Verankerungselementen aufweist, wird durch Einschlagen des Spreizkörpers insbesondere an seinem distalen Ende so weit aufgespreizt, daß der Grundkörper im wesentlichen zylindrische Kontur annimmt. Die Verankerungselemente der aufgespreizten Bereiche dringen dabei in den umliegenden Knochen ein und dienen somit der zusätzlichen Fixierung.

Dieses Fixationselement weist jedoch den Nachteil auf, daß es aufgrund des Absatzes am proximalen Ende nur für die Fixation von Knochenfragmenten bis hin zu einer bestimmten maximalen Abmessung in Längsrichtung des Fixationskörpers geeignet ist. Wird diese maximale Abmessung überschritten, ist keine ausreichende Fixierung des ersten Knochenfragmentes am zweiten Knochenfragment mehr sichergestellt. Da das distale Ende des Fixationskörpers der Spongiosa aufgespreizt wird, die in der Regel geringe Festigkeit aufweist, kann es bei entsprechend geringer Eindringtiefe oder lokal herabgesetzter Festigkeit des Knochengewebes aus dem zweiten Knochenfragment ausreißen. Gerade eine lokal herabgesetzter Festigkeit des Knochengewebes ist während der Operation nur schwer festzustellen, so daß es bei im Normalfall möglicherweise noch ausreichender Eindringtiefe dennoch zum Ausreißen kommen kann.

Fehlt der Absatz am proximalen Ende des Fixationskörpers, so kann der Fixationskörper zwar unabhängig von der Abmessung des ersten Knochenfragmentes ausreichend weit in das zweite Knochenfragment eindringen. Da der Spreizkörper am proximalen Ende einen geringeren Durchmesser als der Hohlraum aufweist, erfolgt unmittelbar am proximalen Ende keine Aufweitung des Fixationskörpers. Die Aufweitung des Fixationskörpers nimmt vielmehr langsam in Richtung des distalen Endes hin zu, so daß mit Fehlen des Absatzes gerade die sichere Fixation relativ dünner erster Knochenfragmente nicht gewährleistet ist.

Das bekannte Fixationselement eignet sich somit in seinen jeweiligen Ausführungen nur bedingt für die Fixation von Knochenfragmenten unterschiedlichster Abmessungen, so daß in der Praxis teurer Satz von Fixationselementen mit einer Vielzahl unterschiedlicher Abmessungen für den jeweiligen Anwendungsfall erforderlich ist.

Aus dem US-Patent 5,713,904 ist ein gattungsgemäßes Fixationselement bekannt, bei dem ein einstückiger rohrförmiger Fixationskörper an beiden Enden mit Längsschlitzen versehen ist, so daß er bei Einführen eines entsprechenden Spreizkörpers in seinen inneren Hohlraum an beiden Enden kelchartig aufgespreizt wird, während er in seinem Mittenbereich unverformt bleibt. Der Fixationskörper ist dabei mit einem Außengewinde versehen, über das er in die Bohrung im Knochen eingeschraubt werden kann.

Der Erfindung liegt deshalb die Aufgabe zugrunde, ein gattungsgemäßes Fixationselement zur Verfügung zu stellen, das die genannten Nachteile nicht oder zumindest in geringerem Maße aufweist, und das insbesondere flexibel einsetzbar ist und eine zuverlässige Fixierung gewährleistet.

Die Aufgabe wird, ausgehend von einem Fixationselement gemäß dem Oberbegriff des Anspruchs 1, durch die im kennzeichnenden Teil des Anspruchs 1 angegebenen Merkmale gelöst.

Die Erfindung schließt die technische Lehre ein, daß man einen flexibel einsetzbares Fixationselement erhält, wenn der Fixationskörper vollständig in die Bohrungen einführbar ausgebildet ist und durch proximales Einführen des Spreizkörpers in den Hohlraum durch Keilwirkung im wesentlichen über seine gesamte Länge zur Verbindung mit dem jeweiligen Knochenfragment quer zu seiner Längsrichtung aufspreizbar ist. Hierdurch ist zum einen sichergestellt, daß der Fixationskörper unabhängig von der Dicke des ersten Knochenfragmentes in eine Position gebracht werden kann, in welcher er der zweite Abschnitt des Fixationskörpers ausreichend tief in das zweite Knochenfragment eingeführt ist, um eine in jedem Fall ausreichende Fixierung des ersten Knochenfragmentes sicherzustellen. Das Aufspreizen des Fixationskörpers im Bereich seines proximalen Endes stellt dabei sicher, daß auch bei besonders dünnen ersten Knochenfragmenten eine zuverlässige Fixierung mit dem erfindungsgemäßen Fixationselement gewährleistet ist.

Die zuverlässige Fixation wird weiterhin dadurch erzielt, daß der Fixationskörper im aufgespreizten Zustand, d. h. nach im wesentlichen vollständigen Einführen des Spreizkörpers in den Hohlraum, am distalen Ende des zweiten Abschnittes eine größere Abmessung quer zu seiner Längsrichtung aufweist als am proximalen Ende des zweiten Abschnittes. Der Fixationskörper ist somit in dem zweiten Knochenfragment, in dem er in der Regel von Spongiosa geringerer Festigkeit umgeben ist, an seinem distalen Ende konisch oder glockenartig aufgeweitet, wodurch eine großflächiger Formschluß erzielt wird. Dieser gewährleistet die sichere Verankerung auch bei möglicherweise herabgesetzter Festigkeit des umliegenden Knochenmaterials, indem er die Verankerungslasten gleichmäßig in ein größeres Knochenvolumen einleitet. Ein Ausreißen des Fixationskörpers aus dem zweiten Knochenfragment ist damit wirkungsvoll vermieden.

Die Gestaltung der für das Aufspreizen des Fixationskörpers verantwortlichen Wirkflächen im Bereich des Hohlraumes und am Spreizelement kann in vielfacher bekannter Weise erfolgen. So ist es für das konische bzw. glockenartige Aufweiten des zweiten Abschnittes des Fixationskörpers lediglich erforderlich, daß die Querabmessung des Hohlraumes zum distalen Ende hin abnimmt. Die Querabmessung des Spreizelements kann dabei zum distalen Ende hin konstant bleiben oder ebenfalls abnehmen, wobei die Abnahme je Längeneinheit dann allerdings geringer sein muß als die Abnahme der Querabmessung des Hohlraumes zum distalen Ende hin.

Der Fixationskörper kann aus einem einzigen Körper bestehen, der zum erleichterten Aufspreizen in den aufzuspreizenden Bereichen mit jeweils einem oder vorzugsweise mehreren Längsschlitzen oder dergleichen versehen ist. Sind mehrere dieser Längsschlitze vorgesehen, wird durch die in Umfangsrichtung variierende Aufspreizung zusätzlich eine Verdrehsicherung um die Längsachse des Fixationselementes erzielt. Vorzugsweise besteht der Fixationskörper aus wenigstens zwei in Umfangsrichtung aneinander anschließenden Teilkörpern, die zum Aufspreizen ausreichend beweglich miteinander verbunden sind. Die Verbindung muß dabei lediglich so fest sein, daß die Teilkörper beim Einführen in die Bohrungen und beim Einführen des Spreizelementes in den Hohlraum zumindest so lange in Längs- und Umfangsrichtung relativ zueinander gehalten werden, bis Spreizelement und Bohrungswand diese Funktion beim Aufspreizen übernehmen. Auch hier wird durch die in Umfangsrichtung variierende Aufspreizung zusätzlich eine Verdrehsicherung um die Längsachse des Fixationselementes erzielt.

Die Verbindung der Teilkörper kann über entsprechend dünne stegartige Überbrückungsbereiche erfolgen, die an den Teilkörpern angeschlossen sind und beim Aufspreizen entsprechend leicht aufgedehnt, aufgefaltet oder aufgerissen werden. Es ist jedoch auch möglich, an den Teilkörpern jeweils Führungselemente vorzusehen, die mit den entsprechenden Führungselementen des angrenzenden Teilkörpers zusammenwirken und so die Teilkörper in Längs- und Umfangsrichtung relativ zueinander halten. So können beispielsweise Vorsprünge mit einer oder mehreren im wesentlichen tangential verlaufenden Führungsflächen an einem Teilkörper mit entsprechend ausgebildeten Führungsnuten am angrenzenden Teilkörper zusammenwirken.

Bei bevorzugten Ausführungen der Erfindung sind die zum Aufspreizen des Fixationskörpers zusammenwirkenden Wirkflächen des Fixationskörpers und des Spreizkörpers derart ausgebildet, daß das Aufspreizen des zweiten Abschnittes am distalen Ende des zweiten Abschnittes beginnt. Hierdurch ist sichergestellt, daß beim Aufspreizen im zweiten Abschnitt eine möglichst gleichmäßige Spannungsverteilung erzielt wird und somit lokale Spannungsspitzen sowohl im Fixationskörper als auch im Knochen im wesentlichen vermieden werden.

Die Gestaltung der für das Aufspreizen des Fixationskörpers verantwortlichen Wirkflächen im Bereich des Hohlraumes und am Spreizelement kann hierzu in vielfacher bekannter Weise erfolgen. So ist es für das am distalen Ende beginnende konische bzw. glockenartige Aufweiten des zweiten Abschnittes des Fixationskörpers lediglich erforderlich, daß die Querabmessungen des Hohlraumes und des Spreizkörpers zum distalen Ende hin abnehmen, wobei die Querabmessung des Hohlraumes und des Spreizkörpers einander am distalen Ende im wesentlichen entsprechen und die Abnahme der Querabmessung des Spreizkörpers je Längeneinheit geringer sein muß als die Abnahme der Querabmessung des Hohlraumes zum distalen Ende hin.

Hierbei ist es besonders vorteilhaft, wenn die Wirkflächen derart ausgebildet sind, daß wenigstens ein erster Teilabschnitt des ersten Abschnitts vor dem Aufspreizen des zweiten Abschnittes aufgespreizt ist. Hierdurch wird die Verkürzung des zweiten Abschnittes infolge dessen am distalen Ende beginnender konischer oder glockenförmiger Aufspreizung über den bereits aufgespreizten ersten Teilabschnitt des ersten Abschnitts auf das erste Knochenfragment übertragen, so daß dieses in für die Heilung vorteilhafter Weise gegen das zweite Knochenfragment gedrückt wird. Weiter vorzugsweise ist dabei der erste Teilabschnitt im Bereich des proximalen Endes des ersten Abschnitts angeordnet, damit dieser Vorteil auch im Falle eines dünnen ersten Knochenfragments genutzt werden kann.

Bei besonders günstigen Weiterbildungen des erfindungsgemäßen Fixationselementes ist der erste Abschnitt an seinem distalen Ende über wenigstens ein Stegelement mit dem proximalen Ende des zweiten Abschnitts schwenkbar verbunden. Dabei sind der Fixationskörper und der Spreizkörper derart ausgebildet, daß bei Einführen des Spreizkörpers das distale Ende des ersten Abschnitts im wesentlichen vollständig aufgespreizt ist, bevor aufeinanderfolgend in einem ersten Schritt ein Teilabschnitt des zweiten Abschnitts und in einem zweiten Schritt das proximale Ende des zweiten Abschnitts aufgespreizt werden. Alternativ können der Fixationskörper und der Spreizkörper derart ausgebildet sein, daß bei Einführen des Spreizkörpers das proximale Ende des zweiten Abschnitts im wesentlichen vollständig aufgespreizt ist, bevor aufeinanderfolgend in einem ersten Schritt ein Teilabschnitt des ersten Abschnitts und in einem zweiten Schritt das distale Ende des ersten Abschnitts aufgespreizt werden. Zudem ist das Stegelement derart ausgebildet und angeordnet, daß sich der Längsabstand zwischen dem ersten und zweiten Abschnitt während des zweiten Schrittes verkürzt.

Hierdurch wird in einfacher Weise erreicht, daß die Verkürzung des Längsabstandes zwischen dem ersten und zweiten Abschnitt über die bei Einsetzen der Verkürzung bereits durch Aufspreizen mit dem jeweiligen Knochenfragment verbundenen Bereiche des ersten bzw. zweiten Abschnittes auf das erste und zweite Knochenfragment übertragen werden. Hierdurch werden die Knochenfragmente nicht nur fest zueinander fixiert, sondern auch in für die Heilung vorteilhafter Weise aufeinandergepreßt.

Das bzw. die Stegelemente können dabei in einfacher Weise so an den beiden Abschnitten angeordnet sein, daß ihr Neigungswinkel zur Längsachse des Fixationskörpers während des zweiten Schrittes zunimmt, woraus sich dann unmittelbar eine entsprechende Verkürzung des Längsabstandes zwischen dem ersten und zweiten Abschnitt ergibt.

Bei vorteilhaften Varianten der Erfindung weist der Fixationskörper nach Einführen des Spreizkörpers in den Hohlraum am proximalen Ende des ersten Abschnittes eine größere Abmessung quer zu seiner Längsrichtung auf als am distalen Ende des ersten Abschnittes. Die hiermit erzielte konische oder glockenförmige Aufweitung am proximalen Ende des Fixationskörpers begünstigt in der schon oben für das distale Ende beschriebenen Weise die schonende Lasteinleitung in das Knochengewebe des betreffenden Knochenfragments.

Das erfindungsgemäße Fixationselement zeichnet sich dadurch aus, daß der Fixationskörper im wesentlichen über seine gesamte Länge aufspreizbar ausgebildet ist. Hierdurch ist eine adäquat verteilte, den Festigkeitsverhältnissen des Knochenmaterials angepaßte Lasteinleitung in das Knochengewebe sichergestellt.

Bei besonders günstigen Varianten des erfindungsgemäßen Fixationselementes weist der erste Abschnitt im nicht aufgespreizten Zustand eine größere Abmessung quer zu seiner Längsrichtung auf als der zweite Abschnitt. Hierbei ist die optimale Positionierung des Fixationskörpers in einfacher und zuverlässiger Weise gewährleistet, indem das zweite Knochenfragment bei der Vorbereitung mit einem entsprechend kleineren Durchmesser aufgebohrt wird als das erste Knochenfragment. Der dadurch entstehende Absatz im Bereich des Bruchspalts bildet dann einen Anschlag für den Fixationskörper, der somit ohne weitere Hilfsmittel unabhängig von der Dicke des ersten Knochenfragmentes mit der optimalen Eindringtiefe in das zweite Knochenfragment eingeführt werden kann.

Das erfindungsgemäße Fixationselement weist zur optimalen Fixierung im Knochengeweben vorzugsweise an der dem Knochen zugewandten Oberfläche des Fixationskörpers Vorsprünge auf, die zum Eindringen in den Knochen vorgesehen sind. Diese Vorsprünge können in vielfacher bekannter Weise ausgebildet sein. Sie können beispielsweise sowohl in Längsrichtung als auch alternativ oder zusätzlich in Umfangsrichtung zahn- oder dornartig oder in anderer Weise ausgebildet sein, um ein widerhakenartiges Einkrallen in das Knochengewebe zu erzielen. Dabei können sie z. B. in Ringen oder gewindeartig am Umfang des Fixationskörpers umlaufend angeordnet sein.

Es versteht sich, daß die Bestandteile des erfindungsgemäßen Fixationselementes aus biokompatiblen Materialien bestehen. Bei besonders vorteilhaften Varianten des erfindungsgemäßen Fixationselementes ist vorgesehen, daß wenigstens der Fixationskörper aus einem bioresorbierbaren Material besteht, so daß sich dessen späteres Explantieren erübrigt. Vorzugsweise sind natürlich sämtliche Bestandteile des Fixationselementes aus derartigen bioresorbierbaren Materialien aufgebaut, um ein späteres Explantieren einzelner Bestandteile vollends zu vermeiden. Dies kann sich natürlich auch durch die Verwendung langzeitbiokompatibler Werkstoffe, beispielsweise für den Spreizkörper, erübrigen. Als Werkstoffe können alle biokompatiblen oder bioresorbierbaren Werkstoffe mit für die Fixation ausreichender Zeitstandfestigkeit verwendet werden, der Fixationskörper besteht vorzugsweise aus einem Polylactid. Dieses ist weiter vorzugsweise in Bereichen mit erhöhter Zugbeanspruchung durch zugfeste, insbesondere bioresorbierbare, Fasern und/oder Fasergewebe verstärkt. Hierbei können beispielsweise zugfeste Werkstoffe verwendet werden, wie sie für chirurgische Nahtmaterialien Anwendung finden. Ein Beispiel hierfür ist das bioresorbierbare Polyglactid.

Andere vorteilhafte Weiterbildungen der Erfindung sind in den Unteransprüchen gekennzeichnet bzw. werden nachstehend zusammen mit der Beschreibung bevorzugter Ausführungen der Erfindung anhand der Figuren näher dargestellt. Es zeigen:
- Figur 1: ein bevorzugtes Ausführungsbeispiel des erfindungsgemäßen Fixationselementes im aufgespreizten Zustand;
- Figur 2: einen Schnitt entlang Linie II-II aus Figur 1;
- Figur 3: einen Axialschnitt durch die Ausführung aus Figur 1 im teilweise aufgespreizten Zustand;
- Figur 4: ein weiteres Ausführungsbeispiel des erfindungsgemäßen Fixationselementes im aufgespreizten Zustand;
- Figur 5: einen Axialschnitt durch die ein anderes Ausführungsbeispiel des erfindungsgemäßen Fixationselementes im teilweise aufgespreizten Zustand.

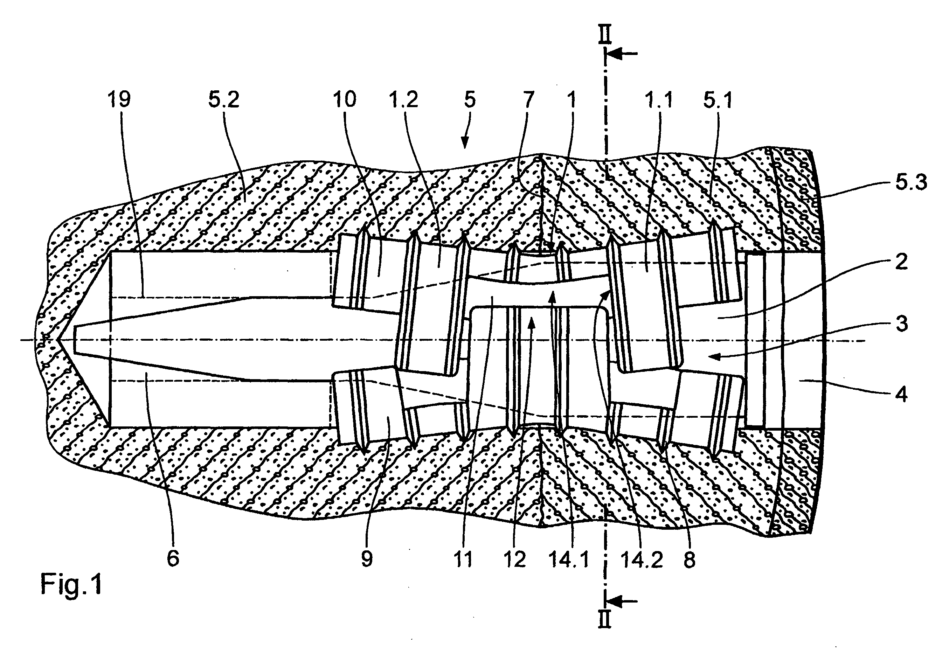

Figur 1 zeigt eine Ausführung des erfindungsgemäßen Fixationselementes mit einem langgestreckten Fixationskörper 1 und einem Spreizkörper 2, der in den sich über die gesamte Länge des Fixationskörpers 1 erstreckenden Hohlraum 3 eingeführt wurde. Der Fixationskörper 1 sitzt mit seinem proximalen, ersten Abschnitt 1.1 in einer Bohrung 4 im ersten Fragment 5.1 des Knochens 5 und mit seinem distalen, zweiten Abschnitt 1.2 in der mit der Bohrung 4 fluchtenden Bohrung 6 im zweiten Fragment 5.2 des Knochens 5. Im gezeigten Beispiel handelt es sich bei dem Knochen 5 um die Tibia im Bereich des oberen Sprunggelenkes, von der im Bereich des Innenknöchels infolge eines Bruches 7 das erste Knochenfragment 5.1 abgetrennt wurde.

Um das erste Knochenfragment 5.1 am zweiten Knochenfragment 5.2 zu fixieren wurde der Fixationskörper zunächst in die reponierten Knochenfragmente 5.1 und 5.2 vollständig bis zur gezeigten Tiefe in die Bohrungen 4 und 6 eingeführt und dann durch Einführen des Spreizkörpers 2 in den Hohlraum 3 durch Keilwirkung über seine gesamte Länge aufgespreizt, wodurch Teile des Fixationskörpers in den umliegenden Knochen 5 eindringen. Hierdurch wird eine feste Verbindung mit den beiden Knochenfragmenten 5.1 und 5.2 und damit die Fixierung der Knochenfragmente 5.1 und 5.2 zueinander erzielt.

Der Spreizkörper 2 und der Hohlraum 3 des Fixationskörpers 1 sind so aufeinander abgestimmt, daß der Fixationskörper 1 am distalen Ende des zweiten Abschnittes 1.2 weiter aufgespreizt ist als am proximalen Ende des zweiten Abschnittes. Das zweiten Knochenfragment 5.2 besteht im Bereich der Bohrung 6 aus spongiösem Knochengewebe, das relativ geringe lokale Festigkeit aufweist. Durch das weitere Aufspreizen am distalen Ende des zweiten Abschnittes 1.2 als am proximalen Ende wird ein Formschluß zwischen Knochen 5 mit einer relativ großen Kontaktfläche erzielt. Diese große Kontaktfläche stellt sicher, daß die Fixationslasten, die über die Kontaktfläche verteilt in den umliegenden Knochen eingeleitet werden, nicht zu lokalen Spannungen im Knochen führen, welche die lokale Festigkeit des Knochens übersteigen und somit zum Lockern der Verbindung bis hin zum Ausreißen bzw. Ausbrechen des Fixationselementes führen können. Hierdurch ergibt sich ein besonders guter, zuverlässiger Halt des Fixationselementes im zweiten Knochenfragment 5.2.

Auch das proximale Ende des ersten Abschnittes 1.1 ist im gezeigten Beispiel weiter aufgespreizt als dessen distales Ende. Hierdurch wird auch bei der Verbindung des Fixationselementes mit dem ersten Knochenfragment 1.1 der eben beschriebene Effekt der gleichmäßigen Lasteinleitung in den Knochen 5 genutzt. Dies ist insbesondere vorteilhaft, wenn es sich bei dem ersten Knochenfragment um ein Fragment handelt, dessen Dicke wie im gezeigten Beispiel die Länge des ersten Abschnittes des Fixationskörpers deutlich übersteigt und damit auch der erste Abschnitt des Fixationskörpers im wesentlichen von Spongiosa geringerer Festigkeit umgeben ist. Es versteht sich jedoch, daß bei langen Ausführungen des Fixationselementes das proximale Ende des ersten Abschnitts auch weniger weit aufgespreizt sein kann, wenn im dieses in der Regel im Bereich der festeren Kortikalis 5.3 liegt.

Der Fixationskörper 1 ist an seinem Umfang mit zahnartigen, im nicht expandierten Zustand ringförmig umlaufenden Vorsprüngen 8 versehen, die beim Aufspreizen in das umgebende Knochengewebe eindringen und eine zusätzliche Fixierung des Fixationskörpers 1 in Axialrichtung bewirken. Es versteht sich, daß diese Vorsprünge bei anderen Varianten der Erfindung auch anders ausgebildet und angeordnet sein können. Um ihren Zweck zu erfüllen, müssen sie lediglich so ausgebildet sein, daß sie in das umliegende Knochengewebe eindringen und mit diesem dann einen Formschluß in Axialrichtung des Fixationskörpers bilden. Es versteht sich weiterhin, daß diese Vorsprünge bei anderen Varianten der Erfindung auch gänzlich fehlen können. Bei diesen Varianten wird dann die Verbindung zwischen Knochen und Fixationselement durch den bei unterschiedlich weiter Aufspreizung an einem oder beiden Enden und der Mitte des Fixationselementes erzielten Formschluß mit dem Knochen und/oder den Kraftschluß zwischen Knochen und Fixationskörper erzielt.

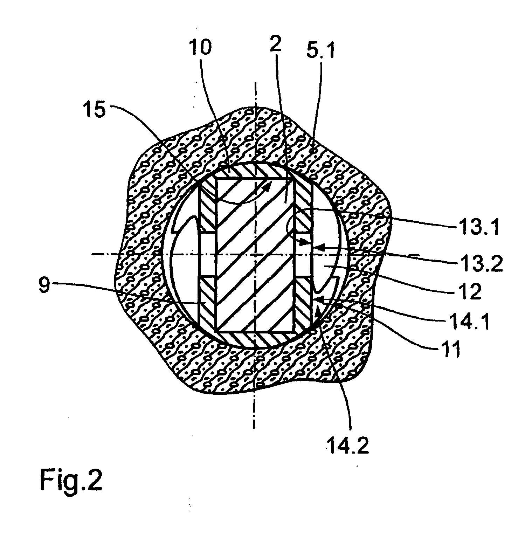

Wie den Figuren 1 und 2 zu entnehmen ist, besteht der Fixationskörper 1 in gezeigten Beispiel aus zwei in Umfangsrichtung aneinander anschließenden, im - in den Figuren nicht dargestellten - nicht aufgespreizten Zustand etwa halbzylindrischen Teilkörpern 9 und 10. Diese sind zum ungestörten Aufspreizen des Fixationskörpers 1 in Radialrichtung über in Führungsnuten 11 des jeweils anderen Teilkörpers 9 bzw. 10 eingreifende Führungsnasen 12 miteinander verbunden. Die Führungsnasen 12 erstrecken sich dabei in Umfangsrichtung und weisen Führungsflächen 13.1, 13.2 auf, die mit entsprechenden Führungsflächen 14.1, 14.2 der Führungsnuten 11 zusammenwirken. Die Führungsflächen 13.1 und 14.1 fixieren dabei die Teilkörper 9 und 10 quer zur Aufspreizrichtung zueinander, während die Führungsflächen 13.2 und 14.2 die Fixierung in Längsrichtung übernehmen. Hierdurch kann der Fixationskörper 1 problemlos im nicht expandierten Zustand in die Bohrungen 4 und 6 eingeführt werden und wird dann definiert aufgespreizt, ohne daß sich die Teilkörper 9,10 in Längsrichtung zueinander verschieben können. Die Fixierung muß dabei selbstverständlich nur so lange bestehen, bis ein ungewolltes Verschieben der Teilkörper 9,10 zueinander durch die in den umliegenden Knochen 4 eindringenden Bereiche der Teilkörper 9, 10 ohnehin verhindert wird.

Es versteht sich jedoch, daß der Fixationskörper bei anderen Varianten des erfindungsgemäßen Fixationselementes auch anders aufgebaut sein kann. So kann er beispielsweise auch aus mehr als zwei Teilkörpern aufgebaut sein. Die Teilkörper können weiterhin auch über entsprechende Stege oder dergleichen einstückig miteinander verbunden sein, wobei die Stege dann das Aufspreizen nicht über Gebühr behindern dürfen. Hierzu können sie so ausgebildet sein, daß sie sich beim Aufspreizen entsprechend dehnen oder auffalten oder aber auch aufreißen. Es versteht sich jedoch auch, daß der Fixationskörper einstückig mit entsprechenden das Aufspreizen ermöglichenden Längsschlitzen oder dergleichen ausgebildet sein kann. Weiterhin versteht es sich, daß auch die Außenkontur des Fixationskörpers nicht notwendigerweise zylindrisch sein muß. Sie kann beispielsweise. auch prismenartig mit beliebiger, z. B. polygonartiger Grundfläche gestaltet sein.

Wie Figur 2 weiterhin zu entnehmen ist, weist der Spreizkörper 2 einen rechteckförmigen Querschnitt auf, der mit entsprechend geformten, den Hohlraum 3 des Fixationskörpers 1 begrenzenden Wirkflächen 15 zusammenwirkt. Es versteht sich allerdings, daß der Spreizkörper bei anderen Varianten auch einen anderen Querschnitt aufweisen kann. So ist beispielsweise ein kreis-, ellipsen- oder polygonförmiger Querschnitt möglich, der dann mit einer entsprechend geformten, den Hohlraum bildenden Nut in dem jeweiligen Teilkörper zusammenwirkt.

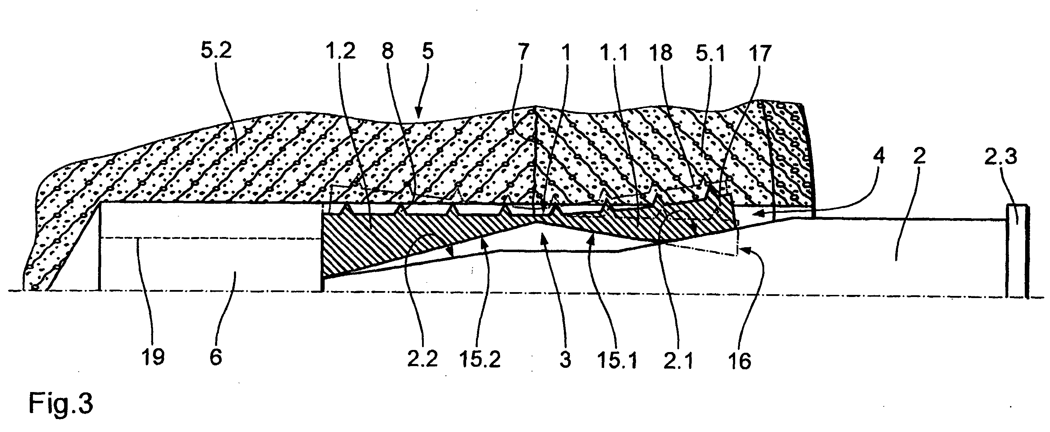

Figur 3 zeigt einen Halbschnitt in Längsrichtung durch die Ausführung aus Figur 1 im teilweise aufgespreizten Zustand. Wie Figur 3 zu entnehmen ist, nimmt die Querabmessung des Hohlraumes 3 von etwa der Mitte des Fixationskörpers 1 zu dessen Enden hin jeweils kontinuierlich ab. Die Querabmessung des Spreizkörpers 2 nimmt demgegenüber in zwei Stufen 2.1 und 2.2 zum distalen Ende hin ab.

Der Spreizkörper 2 weist dabei an seinem vorlaufenden, distalen Ende eine Querabmessung auf, die im nicht aufgespreizten Zustand etwa der Querabmessung der den Hohlraumes 3 begrenzenden Wirkfläche 15.2 am distalen Ende des zweiten Abschnittes 1.2 entspricht. Weiterhin nimmt die Querabmessung des Hohlraumes 3 vom distalen Ende des zweiten Abschnittes 1.2 nach proximal je Längeneinheit stärker zu als die Querabmessung der zweiten Stufe 2.2 des Spreizkörpers 2 von dessen distalem Ende her. Hierdurch wird erreicht, daß das Aufspreizen des zweiten Abschnittes 1.2 des Fixationskörpers 1 von dessen distalem Ende her beginnt. Dies bewirkt, daß beim Aufspreizen im zweiten Abschnitt eine möglichst gleichmäßige Spannungsverteilung erzielt wird und somit lokale Spannungsspitzen sowohl im Fixationskörper als auch im Knochen im wesentlichen vermieden werden.

Der Spreizkörper 2 weist weiterhin am distalen Ende der ersten Stufe 2.1 eine Querabmessung auf, die im nicht aufgespreizten Zustand - wie in Figur 3 durch die strichpunktierte Kontur 16 angedeutet - etwa der Querabmessung der den Hohlraum 3 begrenzenden Wirkfläche 15.1 am proximalen Ende des ersten Abschnittes 1.1 entspricht. Weiterhin ist der Längsabstand zwischen dem distalen Ende der ersten Stufe 2.1 und dem distalen Ende der zweiten Stufe 2.2 geringer als der Längsabstand zwischen dem distalen Ende des zweiten Abschnitts 1.2 und dem proximalen Ende des ersten Abschnitts 1.1. Hierdurch wird erreicht, daß der am proximalen Ende gelegene erste Teilabschnitt 16 des ersten Abschnitts 1.1 vordem Aufspreizen des zweiten Abschnittes 1.2 aufgespreizt wird. Mit anderen Worten ist der Fixationskörper 1 bereits an seinem proximalen Ende im ersten Knochenfragment 5.1 verankert, bevor die Verankerung im zweiten Knochenfragment 5.2 erfolgt.

Durch die ungleichmäßige Aufspreizung verkürzt sich der Fixationskörper 1 - wie in Figur 3 durch die strichzweipunktierte Kontur 18 angedeutet - in seiner Längsrichtung. Die Verkürzung des zweiten Abschnittes 1.2 infolge dessen am distalen Ende beginnender Aufspreizung wird über den bereits aufgespreizten ersten Teilabschnitt 16 des ersten Abschnitts auf das erste Knochenfragment 5.1 übertragen, so daß dieses in für die Heilung vorteilhafter Weise gegen das zweite Knochenfragment 5.2 gedrückt wird.

Wie in Figur 1 und 3 durch die Kontur 19 angedeutet, kann in den Grund der Bohrung 6 vor Einführen des Fixationskörpers 1 eine entsprechend lange Distanzhülse eingebracht werden, die beim Einführen des Spreizkörpers 2 in den Hohlraum 3 zumindest so lange ein Verschieben des Fixationskörpers 1 nach distal verhindert, bis der Fixationskörper 1 infolge des Aufspreizens ausreichend im umliegenden Knochen 5 verankert ist. Es versteht sich, daß hierfür auch andere Hilfsmittel verwendet werden können bzw. sich solche Hilfsmittel erübrigen, wenn die bis zur Verankerung des Fixationskörpers 1 infolge des Aufspreizens im umliegenden Knochen 5 auftretenden Längskräfte die zur Verschiebung des Fixationskörpers 1 im jeweiligen Aufspreizzustand nicht überschreiten.

An seinem proximalen Ende weist der Spreizkörper 2 weiterhin einen Vorsprung 2.3 auf, der das weitere Einführen des Spreizkörpers 2 in den Fixationskörper 1 bei Erreichen einer vorgegebenen Endlage verhindert und somit bei jeder Anwendung ein genau definiertes Aufspreizen sicherstellt.

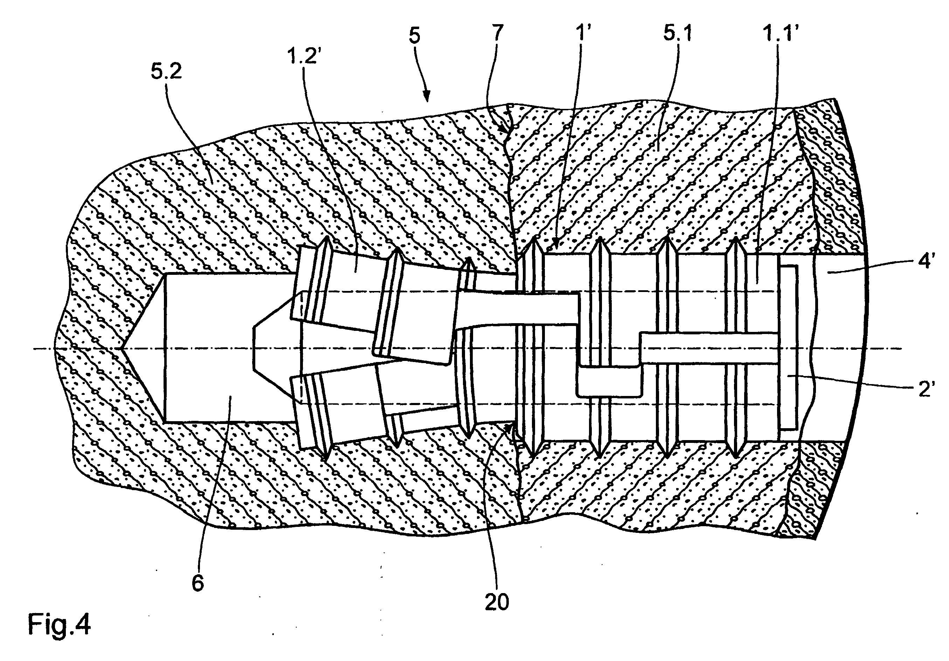

Figur 4 zeigt eine weitere bevorzugte Ausführung des erfindungsgemäßen Fixationselementes, das in seinem grundsätzlichen Aufbau der Variante aus Figur 1 entspricht, so daß hier lediglich auf die Unterschiede eingegangen werden soll. Einer dieser Unterschiede besteht darin, daß der erste Abschnitt 1.1' des Fixationskörpers 1' eine größere Querabmessung aufweist als der zweite Abschnitt 1.2' des Fixationskörpers 1'. Dementsprechend ist auch der Durchmesser der Bohrung 4' im ersten Knochenfragment 5.1 entsprechend größer als der Durchmesser der Bohrung 6' im zweiten Knochenfragment 5.2. Der so entstehende Absatz 20 im Bereich des Frakturspaltes 7 dient somit als Anschlag für den Fixationskörper 1' bei dessen Einführen in die Bohrungen 4', 6' im nicht aufgespreizten Zustand. Hierdurch ist in einfacher Weise sichergestellt, daß der Fixationskörper 1' zum einen unabhängig von der Dicke des ersten Knochenfragmentes 5.1 stets in der optimalen Lage zum Bruchspalt 7 angeordnet ist, und daß zum anderen kein Verschieben des Fixationskörpers 1' aus dieser optimalen Position durch beim Einführen des Spreizelementes 2' wirkende Längskräfte erfolgen kann.

Der erste Abschnitt 1.1' des Fixationskörpers 1' ist im gezeigten Beispiel gleichmäßig aufgespreizt, während die Aufspreizung im zweiten Abschnitt 1.2' zum distalen Ende hin zunimmt. Der Spreizkörper 2' weist hierzu abgesehen von einem abgeschrägten distalen Ende eine im wesentlichen konstante Querabmessung auf, gleiches gilt für den Hohlraum 3' des ersten Abschnitts 1.1' des Fixationskörpers 1', während die Querabmessung des Hohlraumes 3' im zweiten Abschnitt 1.2' im nicht aufgespreizten Zustand nach distal hin abnimmt. Es versteht sich jedoch, daß bei anderen Varianten der Erfindung das Aufspreizen des Fixationskörpers auch bei ersten und zweiten Abschnitten unterschiedlicher Querabmessungen ähnlich wie bei der Ausführung aus Figur 1 erfolgen kann.

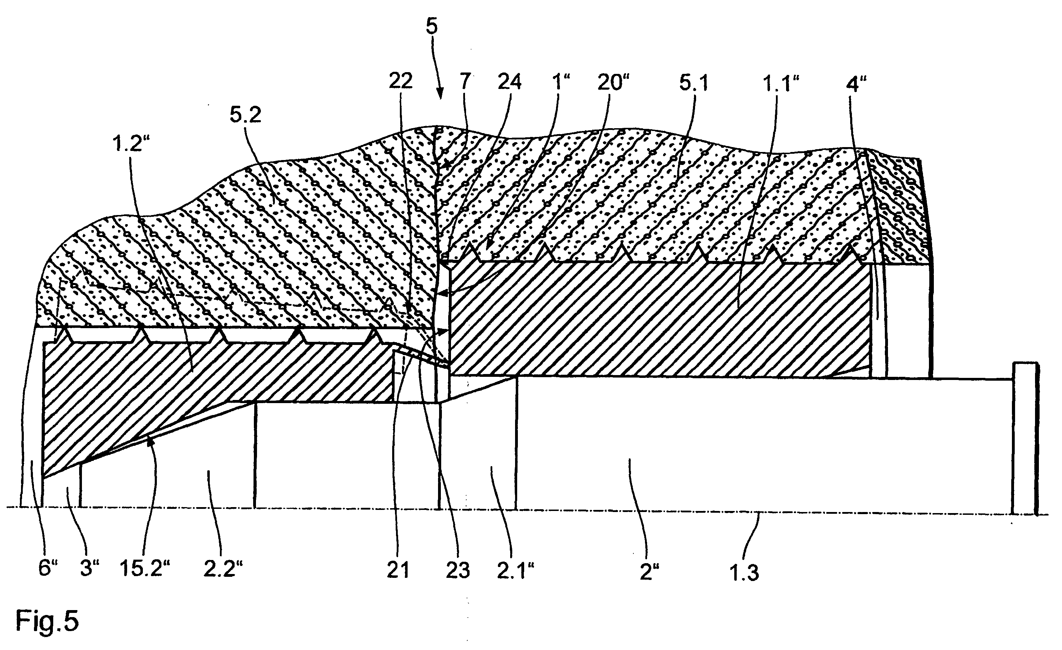

Figur 5 zeigt einen Halbschnitt in Längsrichtung durch eine weitere Ausführung des erfindungsgemäßen Fixationselementes im teilweise aufgespreizten Zustand. Der Aufbau entspricht grundsätzlich der Variante aus Figur 1, so daß hier lediglich auf die Unterschiede eingegangen werden soll.

Bei dieser Variante ist der erste Abschnitt 1.1" über einen in Umfangsrichtung verlaufenden Steg 21 einstückig mit dem zweiten Abschnitt 1.2" des Fixationskörpers 2" verbunden. Im gezeigten Zustand berührt das distale Ende des über seine gesamte Länge kreisförmigen Querschnitt aufweisenden Spreizkörpers 2" gerade die den Hohlraum 3" begrenzende Wirkfläche 15.2", d. h. bei weiterem Vortreiben des Spreizkörpers 2" nach distal beginnt das Aufspreizen des zweiten Abschnitts 1.2" des Fixationskörpers 2". Die zweite Stufe 2.2" des Spreizkörpers 2" und die Wirkfläche 15.2" des zweiten Abschnitts 1.2" sind dabei, wie bereits zu Figur 3 ausführlich beschrieben, so aufeinander abgestimmt, daß das Aufspreizen des zweiten Abschnitts 1.2" in einem ersten Schritt an dessen distalem Ende erfolgt, bevor in einem zweiten Schritt das proximale Ende des zweiten Abschnitts 1.2" aufgespreizt wird. Der erste Abschnitt 1.1" ist im gezeigten Zustand aufgrund des geringen Längsabstandes zwischen der ersten Stufe 2.1 " und der zweiten Stufe 2.2" des Spreizkörpers 2" bereits vollständig aufgespreizt.

Der Steg 21 ist dabei so am ersten und zweiten Abschnitt 1.1" und 1.2" angeordnet, daß sein Neigungswinkel zur Längsachse 1.3 des Fixationskörpers während des zweiten Schrittes zunimmt - wie dies in Figur 5 durch die Kontur 22 angedeutet ist. Hieraus ergibt sich eine Verkürzung des Längsabstandes zwischen dem ersten und zweiten Abschnitt 1.1" und 1.2". Die Verkürzung des Längsabstandes zwischen dem ersten und zweiten Abschnitt 1.1" und 1.2" wird über die bei Einsetzen der Verkürzung bereits durch Aufspreizen mit dem jeweiligen Knochenfragment 5.1 bzw. 5.2 verbundenen Bereiche des ersten bzw. zweiten Abschnittes 1.1" und 1.2" auf das erste und zweite Knochenfragment 5.1 bzw. 5.2 übertragen. Hierbei werden die Knochenfragmente 5.1 und 5.2 aufeinandergepreßt, sofern die resultierende Verkürzung des Längsabstandes zwischen dem ersten und zweiten Abschnitt 1.1" und 1.2" größer ist als der anfängliche Bruchspalt 7 und die Anpreßkraft aus der entsprechenden elastischen Verlängerung des Steges 21 resultiert.

Im gezeigten Beispiel ist wiederum im nicht aufgespreizten Zustand die Querabmessung des ersten Abschnittes 1.1" größer als die Querabmessung des zweiten Abschnittes 1.2", so daß der Fixationskörper 1" definiert bist zum Absatz 20" in die entsprechenden Bohrungen 4" und 6" eingeführt werden kann. In diesem Fall liegt der die distale Endfläche 23 des ersten Abschnitts 1.1 " am zweiten Knochenfragment 5.2 an. Um sicherzustellen, daß nach dem zweiten Schritt auch tatsächlich das erste und zweite Knochenfragment 5.1 und 5.2 aufeinandergedrückt werden und nicht nur die distale Endfläche 23 des ersten Abschnitts 1.1" gegen das zweite Knochenfragment 5.2 gedrückt wird, ist am ersten Abschnitt 1.1" ein Distanzelement 24 vorgesehen. Dieses Distanzelement erstreckt sich in Axialrichtung in einem Abstand zur Längsachse 1.3, der über dem Durchmesser der Bohrung 6" im zweiten Knochenfragment 5.2 liegt. Das Distanzelement 24 ist dabei so ausgebildet, daß es während des zweiten Schrittes leicht in das zweite Knochenfragment 5.2 eindringen kann. Hierdurch ist gewährleistet, daß nach dem zweiten Schritt tatsächlich auch das erste und zweite Knochenfragment 5.1 und 5.2 aufeinandergedrückt werden.

Im gezeigten Beispiel ist nur ein Distanzelement 24 vorgesehen, es versteht sich jedoch, daß bei anderen Varianten der Erfindung auch mehrere über den Umfang verteilte Distanzelemente bzw. ein als umlaufender Steg ausgebildetes Distanzelement vorgesehen sein können, um insbesondere bei Frakturen, die schräg zur Bohrungsachse verlaufen, die geschilderte Wirkung sicherzustellen.

Die in den Figuren 1, 4 und 5 gezeigten Fixationselemente bestehen in allen Bestandteilen jeweils aus einem bioresorbierbaren Material, so daß sich dessen späteres Explantieren erübrigt. Der Fixationskörper besteht dabei aus einem Polylactid. Bei der Variante aus Figur 5 ist das Material im Bereich der Stegs 21 durch ein zugfestes, bioresorbierbares Fasergewebe verstärkt. Hierbei wird bioresorbierbares Polyglactid verwendet, weiches auch sie für chirurgische Nahtmaterialien Anwendung findet.

## Patentansprüche

1. Fixationselement zur Befestigung eines ersten Knochenfragments (5.1), insbesondere eines Knöchelfragments bei Sprunggelenksfrakturen, an einem zugehörigen zweiten Knochenfragment (5.2), das einen langgestreckten Spreizkörper (2; 2'; 2") und einen in fluchtende Bohrungen (4, 6; 4', 6'; 4", 6") in den Knochenfragmenten (5.1, 5.2) einführbaren, langgestreckten Fixationskörper (1; 1'; 1") umfaßt, der einen proximalen, in das erste Knochenfragment (5.1) einzuführenden ersten Abschnitt (1.1; 1.1'; 1.1"), einen daran anschließenden distalen, in das zweite Knochenfragment (5.2) einzuführenden zweiten Abschnitt (1.2; 1.2'; 1.2") sowie einen sich im wesentlichen über seine Länge erstreckenden Hohlraum (3; 3'; 3") aufweist, wobei der Fixationskörper (1; 1'; 1") vollständig in die Bohrungen (4, 6; 4', 6'; 4", 6") einführbar ausgebildet ist, durch proximales Einführen des Spreizkörpers (2; 2'; 2") in den Hohlraum (3; 3'; 3") durch Keilwirkung wenigstens im Bereich seiner beiden Enden zur Verbindung mit dem jeweiligen Knochenfragment (5.1, 5.2) quer zu seiner Längsrichtung aufspreizbar ist und nach im wesentlichen vollständigen Einführen des Spreizkörpers (2; 2'; 2") in den Hohlraum (3; 3'; 3") am distalen Ende des zweiten Abschnittes (1.2; 1.2'; 1.2") eine größere Abmessung quer zu seiner Längsrichtung aufweist als am proximalen Ende des zweiten Abschnittes (1.2; 1.2'; 1.2"), **dadurch gekennzeichnet, daß** der Fixationskörper (1;1'; 1") im wesentlichen über seine gesamte Länge aufspreizbar ist.

2. Fixationselement nach Anspruch 1, **dadurch gekennzeichnet, daß** der Fixationskörper (1; 1'; 1") aus wenigstens zwei in Umfangsrichtung aneinander anschließenden Teilkörpern (9,10) besteht, die zum Aufspreizen ausreichend beweglich miteinander verbunden sind.

3. Fixationselement nach Anspruch 1 oder 2, **dadurch gekennzeichnet, daß** die zum Aufspreizen des Fixationskörpers (1; 1'; 1") zusammenwirkenden Wirkflächen des Fixationskörpers (1; 1'; 1") und des Spreizkörpers (2; 2'; 2") derart ausgebildet sind, daß das Aufspreizen des zweiten Abschnittes (1.2; 1.2'; 1.2") am distalen Ende des zweiten Abschnittes (1.2; 1.2'; 1.2") beginnt.

4. Fixationselement nach Anspruch 3, **dadurch gekennzeichnet, daß** die zusammenwirkenden Wirkflächen des Fixationskörpers (1; 1'; 1") und des Spreizkörpers (2; 2'; 2") derart ausgebildet sind, daß wenigstens ein erster Teilabschnitt (17) des ersten Abschnitts (1.1; 1.1'; 1.1") vor dem Aufspreizen des zweiten Abschnittes (1.2; 1.2'; 1.2") aufgespreizt ist.

5. Fixationselement nach Anspruch 4, **dadurch gekennzeichnet, daß** der erste Teilabschnitt (17) im Bereich des proximalen Endes des ersten Abschnitts (1.1) angeordnet ist.

6. Fixationselement nach einem der vorhergehenden Ansprüche, **dadurch gekennzeichnet**, daßder erste Abschnitt (1.1") an seinem distalen Ende über wenigstens ein Stegelement (21) mit dem proximalen Ende des zweiten Abschnitts (1.2") schwenkbar verbunden ist, wobei
der Fixationskörper (1") und der Spreizkörper (2") derart ausgebildet sind, daß bei Einführen des Spreizkörpers (2") das distale Ende des ersten Abschnitts (1.1") im wesentlichen vollständig aufgespreizt ist, bevor aufeinanderfolgend in einem ersten Schritt ein Teilabschnitt des zweiten Abschnitts (1.2) und in einem zweiten Schritt das proximale Ende des zweiten Abschnitts (1.2") aufgespreizt werden, oder daß bei Einführen des Spreizkörpers (2") das proximale Ende des zweiten Abschnitts im wesentlichen vollständig aufgespreizt ist, bevor aufeinanderfolgend in einem ersten Schritt ein Teilabschnitt des ersten Abschnitts und in einem zweiten Schritt das distale Ende des ersten Abschnitts aufgespreizt werden, und
das Stegelement (21) derart ausgebildet und angeordnet ist, daß sich der Längsabstand zwischen dem ersten und zweiten Abschnitt (1.1", 1.2") während des zweiten Schrittes verkürzt.

7. Fixationselement nach einem der vorhergehenden Ansprüche, **dadurch gekennzeichnet, daß** der Fixationskörper (1) nach Einführen des Spreizkörpers (2) in den Hohlraum (3) am proximalen Ende des ersten Abschnittes (1.1) eine größere Abmessung quer zu seiner Längsrichtung aufweist als am distalen Ende des ersten Abschnittes (1.2).

8. Fixationselement nach einem der vorhergehenden Ansprüche, **dadurch gekennzeichnet, daß** der erste Abschnitt (1.1': 1.1") im nicht aufgespreizten Zustand eine größere Abmessung quer zu seiner Längsrichtung aufweist als der zweite Abschnitt (1.2'; 1.2").

9. Fixationselement nach einem der vorhergehenden Ansprüche, **dadurch gekennzeichnet, daß** an der dem Knochen (5) zugewandten Oberfläche des Fixationskörpers (1; 1'; 1") zum Eindringen in den Knochen (5) vorgesehene Vorsprünge (8) angeordnet sind.

10. Fixationselement nach einem der vorhergehenden Ansprüche, **dadurch gekennzeichnet, daß** wenigstens der Fixationskörper (1; 1'; 1") aus einem bioresorbierbaren Material besteht.

11. Fixationselement nach Anspruch 10, **dadurch gekennzeichnet, daß** der Fixationskörper (1;1'; 1") aus einem Polylactid besteht, das in Bereichen mit Zugbeanspruchung durch zugfeste, insbesondere bioresorbierbare, Fasern und/oder Fasergewebe verstärkt ist.

## Claims

1. A fixation element for the attachment of a first bone fragment (5.1), in particular an anklebone fragment in the case of anklebone fractures, to an associated second bone fragment (5.2), which comprises an elongate expansion member (2; 2'; 2") and an elongate fixation member (1; 1'; 1"), which can be introduced into aligning bores (4, 6; 4', 6'; 4", 6") in the bone fragments (5.1, 5.2) and which comprises a proximal first portion (1.1; 1.1'; 1.1") to be introduced into the first bone fragment (5.1), a connected distal second portion (1.2; 1.2'; 1.2") to be introduced into the second bone fragment (5.2) and also a cavity (3; 3'; 3") extending substantially over its length,
wherein the fixation member (1; 1'; 1") is constructed to be introduced fully into the bores (4, 6; 4', 6'; 4", 6"), by the proximal introduction of the expansion member (2; 2'; 2") into the cavity (3; 3'; 3") can be expanded by a wedging effect at least in the region of its two ends for connection with the respective bone fragment (5.1, 5.2) at right angles to its longitudinal direction and, after substantially complete introduction of the expansion member (2; 2'; 2") into the cavity (3; 3'; 3"), at the distal end of the second portion (1.2; 1.2'; 1.2") has a larger dimension at right angles to its longitudinal direction than at the proximal end of the second portion (1.2; 1.2'; 1.2"),
**characterised in that** the fixation member (1; 1'; 1") can be expanded substantially over its entire length.

2. A fixation element according to Claim 1,
**characterised in that** the fixation member (1; 1'; 1") consists of at least two sub-members (9, 10) connected to one another in the circumferential direction, which are connected to one another with sufficient mobility for expansion.

3. A fixation element according to Claim 1 or 2,
**characterised in that** the effective surfaces of the fixation element (1; 1'; 1") and of the expansion member (2; 2'; 2") which interact for the expansion of the fixation member (1; 1'; 1") are constructed in such a manner that the expansion of the second portion (1.2; 1.2'; 1.2") begins at the distal end of the second portion (1.2; 1.2'; 1.2").

4. A fixation element according to Claim 3,
**characterised in that** the interacting effective surfaces of the fixation member (1; 1'; 1") and of the expansion member (2; 2'; 2") are constructed in such a manner that at least a first sub-portion (17) of the first portion (1.1; 1.1'; 1.1") is expanded before the expansion of the second portion (1.2; 1.2'; 1.2").

5. A fixation element according to Claim 4,
**characterised in that** the first sub-portion (17) is disposed in the region of the proximal end of the first portion (1.1).

6. A fixation element according to one of the preceding Claims,
**characterised in that** at its distal end the first portion (1.1") is swivellably connected via at least one web element (21) to the proximal end of the second portion (1.2"), wherein
the fixation member (1") and the expansion member (2") are constructed so that upon the introduction of the expansion member (2") the distal end of the first portion (1.1") is substantially completely expanded before successively in a first step a sub-portion of the second portion (1.2) and in a second step the proximal end of the second portion (1.2") are expanded, or that upon the introduction of the expansion member (2") the proximal end of the second portion is substantially completely expanded before successively in a first step a sub-portion of the first portion and in a second step the distal end of the first portion are expanded, and
t he web element (21) is constructed and disposed in such a manner that the longitudinal distance between the first and second portions (1.1", 1.2") is reduced during the second step.

7. A fixation element according to one of the preceding Claims,
**characterised in that** after the introduction of the expansion member (2) into the cavity (3), at the proximal end of the first portion (1.1) the fixation member (1) has a greater dimension at right angles to its longitudinal direction than at the distal end of the first portion (1.2).

8. A fixation element according to one of the preceding Claims,
**characterised in that** in the non-expanded state the first portion (1.1'; 1.1") has a greater dimension at right angles to its longitudinal direction than the second portion (1.2'; 1.2").

9. A fixation element according to one of the preceding Claims,
**characterised in that** projections (8) provided for penetration into the bone (5) are disposed on the surface of the fixation member (1; 1'; 1") facing the bone (5).

10. A fixation element according to one of the preceding Claims,
**characterised in that** at least the fixation member (1; 1'; 1") is made from a bioreabsorbable material.

11. A fixation element according to Claim 10,
**characterised in that** the fixation member (1; 1'; 1") is made from a polyactide which is reinforced in regions with tensile stress by high tensile strength, in particular bioreabsorbable, fibres and or fibrous tissue.

## Revendications

1. Elément de fixation destiné à la fixation d'un premier fragment osseux (5.1), en particulier d'un fragment de cheville lors de fractures de la cheville, à un second fragment osseux correspondant (5.2), qui comprend un corps expansible allongé (2 ; 2' ; 2'') et un corps de fixation allongé (1 ; 1' ; 1") insérable dans des orifices alignés (4, 6 ; 4', 6' ; 4', 6'') dans les fragments osseux (5.1, 5.2), qui présente un premier segment (1.1 ; 1.1' ; 1.1'') proximal à insérer dans le premier fragment osseux (5.1), un second segment (1.2 ; 1.2' ; 1.2'') distal, contigu au premier, à insérer dans le second fragment osseux (5.2), ainsi qu'une cavité (3 ; 3' ; 3'') s'étendant essentiellement sur sa longueur, moyennant quoi le corps de fixation (1 ; 1' ; 1'') est configuré pour être entièrement insérable dans les orifices (4, 6 ; 4', 6' ; 4', 6''), est déployable par l'insertion proximale du corps expansible (2 ; 2' ; 2'') dans la cavité (3 ; 3' ; 3'') par clavetage au moins dans la zone de ses deux extrémités en connexion avec le fragment osseux respectif (5.1, 5.2) de travers par rapport à son sens longitudinal et présente, après l'insertion essentiellement intégrale du corps expansible (2 ; 2' ; 2'') dans la cavité (3 ; 3' ; 3'') à l'extrémité distale du second segment (1.2 ; 1.2' ; 1.2''), une dimension supérieure en travers par rapport à son sens longitudinal à celle à l'extrémité proximale du second segment (1.2 ; 1.2' ; 1.2''), **caractérisé en ce que** le corps de fixation (1 ; 1' ; 1'') est déployable essentiellement sur toute sa longueur.

2. Elément de fixation selon la revendication 1, **caractérisé en ce que** le corps de fixation (1 ; 1' ; 1'') se compose d'au moins deux corps partiels (9, 10) rattachés l'un à l'autre dans le sens périphérique, qui sont reliés l'un à l'autre de manière suffisamment amovible pour se déployer.

3. Elément de fixation selon la revendication 1 ou 2, **caractérisé en ce que** les surfaces actives du corps de fixation (1 ; 1' ; 1'') et du corps expansible (2 ; 2' ; 2'') agissant conjointement afin de déployer le corps de fixation (1 ; 1' ; 1'') sont conçues de telle sorte que le déploiement du second segment (1.2 ; 1.2' ; 1.2'') commence à l'extrémité distale du second segment (1.2 ; 1.2' ; 1.2'').

4. Elément de fixation selon la revendication 3, **caractérisé en ce que** les surfaces actives du corps de fixation (1 ; 1' ; 1") et du corps expansible (2 ; 2' ; 2'') agissant conjointement sont conçues de telle sorte qu'au moins un segment partiel (17) du premier segment (1.1 ; 1.1' ; 1.1'') est déployé avant le déploiement du second segment (1.2 ; 1.2' ; 1.2'').

5. Elément de fixation selon la revendication 4, **caractérisé en ce que** le premier segment partiel (17) est disposé dans la zone de l'extrémité proximale du premier segment (1.1).

6. Elément de fixation selon l'une quelconque des revendications précédentes, **caractérisé en ce que** le premier segment (1.1'') est relié de manière pivotante, au niveau de son extrémité distale par au moins un élément de traverse (21), avec l'extrémité proximale du second segment (1.2), moyennant quoi
le corps de fixation (1'') et le corps expansible (2'') sont conçus de telle sorte que, lors de l'insertion du corps expansible (2''), l'extrémité. distale du premier segment (1.1'') est déployée de manière essentiellement intégrale, avant que successivement, dans une première étape, un segment partiel du second segment (1.2) et dans une seconde étape, l'extrémité proximale du second segment (1.2'') soient déployés, ou de telle sorte que, lors de l'insertion du corps expansible (2''), l'extrémité proximale du second segment est déployée de manière essentiellement intégrale, avant que successivement, dans une première étape, un segment partiel du premier segment et dans une seconde étape, l'extrémité distale du second segment (1.2'') soient déployés, et
l'élément de traverse (21) est conçu et disposé de telle sorte que l'écart longitudinal entre le premier et le second segment (1.1'', 1.2'') se raccourcit pendant la seconde étape.

7. Elément de fixation selon l'une quelconque des revendications précédentes, **caractérisé en ce que** le corps de fixation (1) après l'insertion du corps expansible (2) dans la cavité (3) à l'extrémité proximale du premier segment (1.1) présente une dimension supérieure en travers par rapport à son sens longitudinal à celle à l'extrémité distale du premier segment (1.2)

8. Elément de fixation selon l'une quelconque des revendications précédentes, **caractérisé en ce que** le premier segment (1.1' ; 1.1'') à l'état non déployé présente une taille supérieure en travers par rapport à son sens longitudinal au second segment (1.2' ; 1.2'').

9. Elément de fixation selon l'une quelconque des revendications précédentes, **caractérisé en ce que** des saillies (8) prévues pour pénétrer dans l'os (5) sont disposées au niveau des surfaces supérieures tournées vers l'os (5) du corps de fixation (1 ; 1' ; 1'').

10. Elément de fixation selon l'une quelconque des revendications précédentes, **caractérisé en ce qu'**au moins le corps de fixation (1 ; 1' ; 1'') se compose d'un matériau biorésorbable.

11. Elément de fixation selon la revendication 10, **caractérisé en ce que** le corps de fixation (1 ; 1' ; 1'') se compose d'un polylactide, qui est renforcé dans les zones d'effort de traction par des fibres et/ou un tissu de fibres résistant à la traction, particulièrement biorésorbables.
